# FASCICULE DE BREVET EUROPEEN

(11) **EP 0 768 887 B1**
(45) Date de publication et mention de la délivrance du brevet: **06.06.2001**
(21) Numéro de dépôt: 96904129.2
(22) Date de dépôt: 13.02.1996
(51) Int. Cl.: A61K 35/78

(54) **COMPOSITIONS POSSEDANT DES PROPRIETES ANTIVIRALES ET PROCEDE D'OBTENTION**
KOMPOSITIONEN MIT ANTIVIRALEN WIRKUNGEN UND VERFAHREN ZUR HERSTELLUNG
COMPOSITIONS HAVING ANTIVIRAL PROPERTIES AND PREPARATION PROCESS

(30) Priorité: 06.03.1995 FR 9502595
(43) Date de publication de la demande: 23.04.1997
(73) Titulaire: Commin, Alix-Roland, F-94400 Vitry sur Seine (FR)
(72) Inventeur: Commin, Alix-Roland, F-94400 Vitry sur Seine (FR)
(86) Numéro de dépôt international: FR9600230
(87) Numéro de publication internationale: WO9627383

(56) Documents cités:
- EP-A- 0 295 955
- WO-A-93/11217
- WO-A-93/11779

## Description

L'invention a pour objet des compositions possédant notamment des propriétés antivirales et, en particulier, antirétrovirales.

La lutte contre les maladies provoquées par les rétrovirus mobilise de nombreuses équipes de chercheurs de par le monde. En particulier, des efforts considérables sont déployés pour lutter contre l'épidémie du SIDA (Syndrome d'Immunodéficience Acquise).
On sait que l'agent responsable du SIDA est un rétrovirus, appelé VIH, pour Virus de l'Immunodéficience Humaine, ou le plus communément HIV, selon sa désignation abrégée en anglais.

Les médicaments antiviraux les plus utilisés sont des dérivés de nucléosides, dont la zidovudine, ou AZT, qui fut le premier antirétroviral disponible. Depuis, l'émergence de mutants résistants a conduit à rechercher de nouveaux traitements.

Les travaux réalisés par l'inventeur sur un mélange d'acide et de produits d'origine naturelle l'ont amené à mettre au point des compositions de grande efficacité pour inactiver le pouvoir infectieux de HIV.

L'invention a donc pour but de fournir de nouvelles compositions antirétrovirales capables.

Elle vise également un procédé d'obtention de telles compositions.

Les compositions selon l'invention sont caractérisées en ce qu'elles sont élaborées à partir d'acide acétique et d'extraits de poudre de noix de coco, de solution de sels minéraux, d'extraits de cactacée, de liliacée, d'anacardiacée, et d'euphorbiacée.

La noix de coco est le fruit du cocotier. Conformément à l'invention, on utilise avantageusement la chair blanche râpée.

Des cactacées convenant particulièrement pour la mise en oeuvre de l'invention comprennent l'opuntia.
On rappelle que l'opuntia est le nom générique de la raquette, du nopal, du figuier de barbarie et autres espèces originaires de l'Amérique tropicale.
On utilise plus spécialement les fruits de la raquette verte.

Des liliacées appropriées dans le cadre de l'invention comprennent la salsepareille. On utilise avec avantage la racine de la salsepareille.

Comme anacardiacée, on a plus particulièrement recours au mombin, et notamment à l'écorce de mombin.

Une euphorbiacée particulièrement préférée est constituée par le ricin. Ses graines, notamment à l'état grillé et sous forme de poudre, sont plus spécialement utilisées selon l'invention.

Des compositions préférées sont élaborées à partir d'acide acétique, de noix de coco râpée, d'eau de mer, de fruits de raquette verte, de racine de salsepareille, d'écorce de mombin, de poudre de graines de ricin grillées.

On utilise avantageusement ces différents ingrédients selon les proportions suivantes:
- acide acétique: de 5 à 10 litres, notamment de 7 à 8 litres;
- poudre de noix de coco (de préférence, chair blanche râpée): équivalent d'environ 10 noix de coco ;
- solution de sels minéraux (notamment sodium, potassium, magnésium, calcium, phosphates, chlorures, sulfates, carbonates et bicarbonates), par exemple eau de mer : 3 à 5 litres, en particulier 4 litres ;
- raquette, notamment fruits de raquette verte râpés : de 10 à 20 fruits correspondant à 1 à 2 kg, notamment 15 fruits environ, correspondant à environ 1,5 kg ;
- racine de salsepareille: 500 g à 1 kg, notamment 750 g environ ;
- écorce de mombin : de 500 g à 1,5 kg, en particulier environ 1 kg ;
- graines de ricin grillées et moulues : de 500 g à 1,5 kg, notamment environ 1 kg.

Selon un autre aspect, les compositions de l'invention sont telles qu'obtenues en laissant macérer de la noix de coco en poudre avec de l'acide acétique, pendant environ 24 heures, en ajoutant au filtrat du mélange de macération les divers ingrédients énumérés ci-dessus, avantageusement selon les proportions indiquées, en portant le mélange résultant à ébullition pendant au moins 1 heure, et en récupérant le filtrat.

L'invention vise également un procédé d'obtention des compositions définies ci-dessus, caractérisé par les étapes précédemment rapportées.

L'étude des propriétés des compositions de l'invention a mis en évidence un effet inhibiteur de l'activité reverse transcriptase de HIV-1 pouvant dépasser 90 % par rapport à l'activité mesurée chez les témoins. On constate également une diminution de l'effet cytopathogène de HIV-1 chez des cellules infectées par ce virus, traitées par des compositions de l'invention.

L'absence de toxicité de ces compositions a été vérifiée sur la souris par injection intra-péritonéale à pH physiologique.

L'invention vise donc la mise à profit des propriétés de ces compositions pour l'élaboration de compositions pharmaceutiques.

Les compositions pharmaceutiques de l'invention sont caractérisées en ce qu'elles renferment une quantité efficace d'au moins une composition telle que définie ci-dessus, en association avec un véhicule pharmaceutique inerte.

Ces compositions sont particulièrement appropriées pour la prévention et le traitement du SIDA, le cas échéant en combinaison avec d'autres médicaments.

Ces compositions sont administrées de préférence par voie injectable sous forme de solutions ou de suspensions injectables isotoniques vis-à-vis du plasma sanguin.

La quantité de principe actif varie suivant l'état du patient, la posologie chez l'homme étant le plus souvent de l'ordre de 1 à 500 mg par jour de principe actif (extrait sec).

L'invention sera illustrée ci-après par un exemple de préparation d'une composition de grande efficacité vis-à-vis du virus HIV-1 et par les résultats d'essais in vitro et in vivo.

### Exemple 1 : Préparation d'une composition selon l'invention

On fait macérer, pendant au moins 24 h, chair blanche, sèche, râpée, 10 noix de coco avec 7 à 8 litres de vinaigre blanc ou d'acide acétique.
Le mélange est ensuite filtré.
On ajoute au filtrat récupéré les ingrédients suivants:
- solution riche en minéraux , comme de l'eau de mer: 4 litres;
- raquette verte râpée (environ 15 fruits) : 1,5 kg
- racine de salsepareille : 750 g
- écorce de mombin : 1 kg
- poudre de graines de ricin grillées : 1 kg

Le mélange ainsi obtenu est porté à ébullition pendant environ 1 h, puis filtré.

### Exemple 2 : Etude de l'inactivation de HIV-1 par la composition de l'exemple 1.

### PROTOCOLE EXPERIMENTAL

### - Inactivation du virus

La suspension de particules virales A dans le milieu de culture RPMI 1640 (HIV-1 III B, activité transcriptase inverse 3,8 x 10⁶ cpm/ml) est incubée avec la composition de l'exemple 1, appelée ci-après "la composition", (V/V), pendant 20 min à 4°C, puis 1 heure à température ambiante, enfin 2 heures à 37°C. En fin d'incubation, le mélange est dilué au 1/10ème, 1/20, 1/40, 1/100, 1/200 dans le milieu de culture RPMI 1640 plus 10 % de sérum de veau foetal (SVF), chauffé au préalable durant 30 min à 56°C.

### - infection

Des culots de 5 x 10⁵ cellules MT4 (cellules humaines d'origine lymphoïde transformées par HTLV-1) sont remis en suspension dans 250 µl des différentes dilutions du mélange virus-composition.

Après 1 heure d'adsorption à température ambiante, avec remise en suspension des cellules toutes les 15 min, le virus non adsorbé est éliminé par centrifugation des cellules. Celles-ci sont lavées deux fois avec du RPMI, puis remises en suspension dans le milieu RPMI 10 % SVF, à la concentration de 2 x 10⁵/ml. Les cellules infectées sont mises en culture dans des plaques de microtitration (Costar 96 godets) à raison de 200 µl/godet.

Toutes les cultures sont faites au moins en double. Les cultures sont diluées avec un volume identique de milieu après 5 jours et 8 jours d'infection.

Une série de cultures témoins sont infectées en parallèle avec la suspension de virus A mélangée (V/V) avec du milieu RPMI. Les incubations et les dilutions sont réalisées strictement dans les mêmes conditions que pour le virus traité avec la composition.

Un contrôle supplémentaire est effectué avec le virus préincubé avec une solution 0,15 M NaCl.

La production de virus a été mesurée par un dosage de l'activité transcriptase inverse associée aux particules virales libérées par les cellules dans le milieu de culture. La transcriptase inverse est également appelée transcriptase reverse ou RT en abrégé.

Le test RT est fait sur 65 µl de milieu selon le protocole décrit par Moog et col., dans Antiviral Research, 24 (1994), 275-288. Les résultats sont exprimés en cpm/essai (tableau, partie inférieure).

La viabilité cellulaire est mesurée par le test MTT décrit par Moog et col., dans la référence donnée ci-dessus (tableau, patie supérieure). L'intensité de la coloration est exprimée en milli DO.

### EXPERIENCE A :

On évalue la production de HIV après infection de cellules MT4 avec du virus préincubé avec la composition. Après 5 ou 7 jours d'infection, les valeurs des RT sont comprises entre 699 et 1538 cpm/essai, alors que, pour des surnageants de cultures non infectées, elles sont de 1400-2100 cpm/essai. Toutes les cultures infectées avec le virus non traité produisent du virus de J5 à J11.

### Expérience B :

On évalue la production de HIV après infection de cellules CEM-SS par HIV-1 LAi

Une suspension de virus B (HIV-1 LAi, activité réverse transcriptase 3 x 10⁶ cpm/ml) est incubée (V/V) avec la composition. Toutes les autres étapes sont celles décrites pour l'expérience faite sur cellules MT4.

Dans ces conditions, les cultures infectées avec le virus témoin (non incubé avec la composition) produisent du virus, mesurable par RT, 11 jours après l'infection.

Dans le cas des cellules infectées avec le virus préincubé avec la composition, les RT sont semblables à celles mesurées sur des surnageants de cultures non infectées (1707-2564 cpm/essai) après 11 jours d'infection.

### EXPERIENCE C :

Le protocole expérimental suivi est identique à celui de l'expérience A. Les mesures faites à J5 et J7 post-infection montrent qu'on ne détecte pas, ou très peu, de réplication du virus lorsqu'il a été préincubé avec la composition (RT des cellules témoins non infectées 972-12192 cpm/essai).

### EXPERIENCE D :

Afin de pouvoir mesurer la production de virus à des temps plus précoces que dans l'expérimentation B, une concentration de virus 10 fois supérieure a été utilisée.

La production de virus après 5 et 8 jours est très fortement inhibée par la préincubation du virus avec la composition.

L'absence d'effet observé en utilisant la solution 0.15 M NaCl indique que l'inhibition du virus est spécifique de la composition.

Ces résultats montrent donc que la préincubation du virus HIV-1 avec la composition de l'invention entraîne une inactivation du pouvoir infectieux des particules virales.

Exemple 3 : Etude de l'effet de la composition de l'exemple 1 sur l'activité réverse transcriptase d'HIV-1

Une suspension de virus HIV-1 a été mélangée (V/V) à différentes dilutions de la composition. La détermination de l'activité réverse transcriptase (RT) associée aux particules virales a été effectuée comme décrit par Moog et al, dans la référence ci-dessus.

Les mesures ont été effectuées sur au moins deux échantillons.

Les résultats obtenus sont rapportés dans le tableau 1.

On constate que l'utilisation de la composition, aux concentrations les plus élevées, inhibe l'activité RT de plus de 90 %.

Une autre série d'expériences, dont les résultats sont donnés dans le tableau 2, confirme les données précédentes.

Ces expériences mettent donc en évidence que les compositions selon l'invention sont capables d'inhiber l'activité de la réverse transcriptase mesurée in vitro.

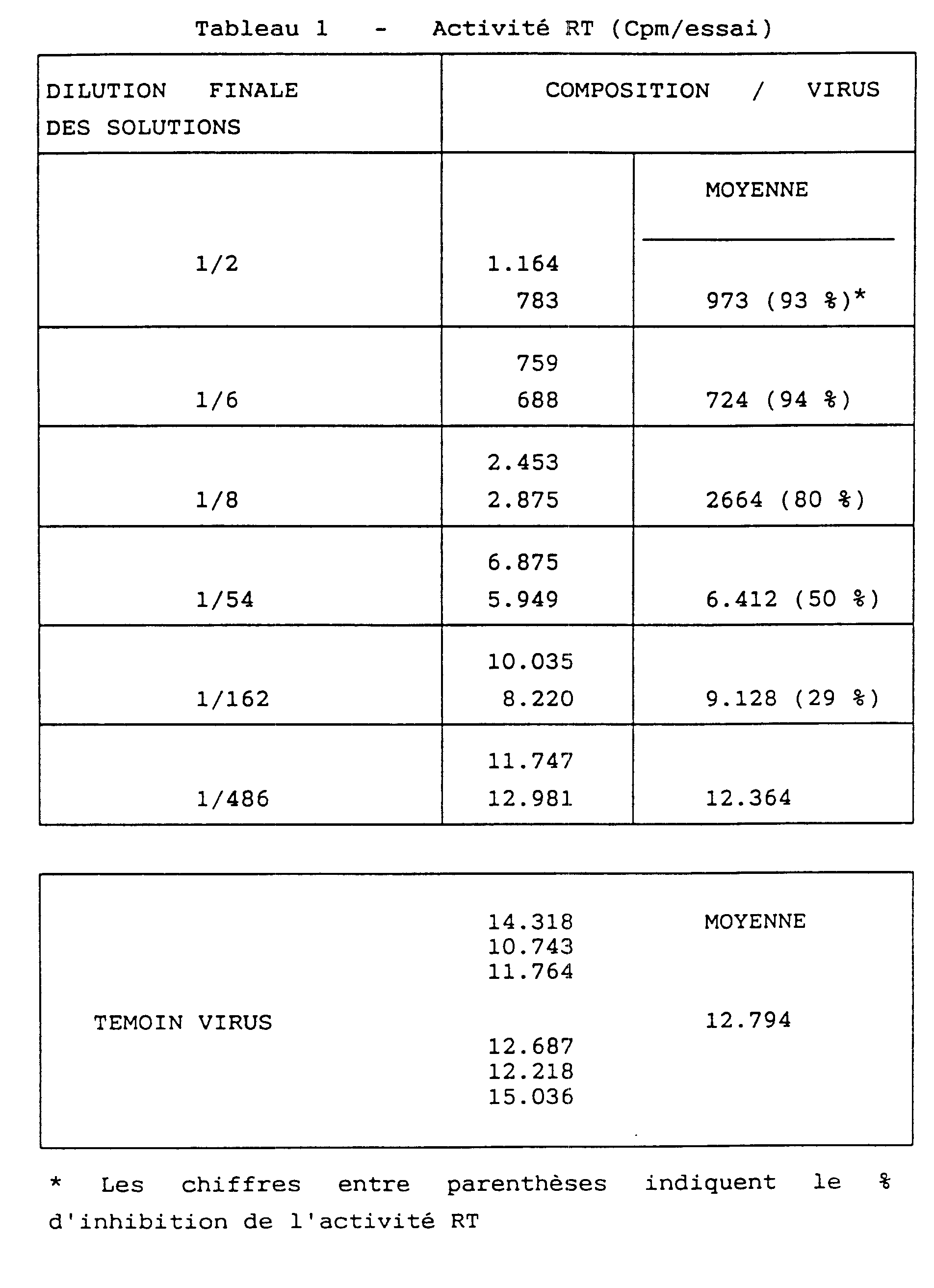

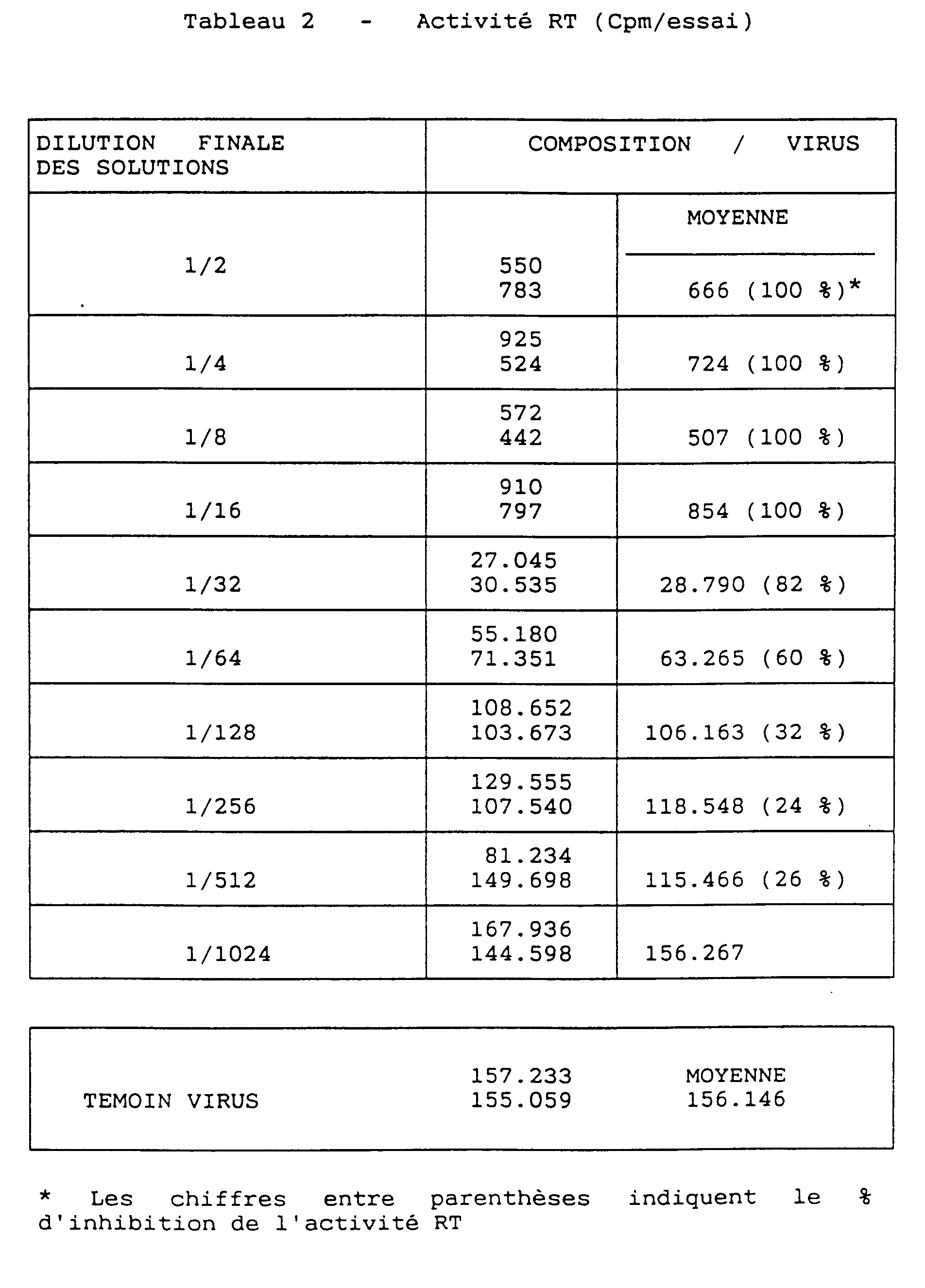

## Revendications

1. Compositions possédant notamment des propriétés antivirales et, en particulier, antirétrovirales, caractérisées en ce qu'elles sont élaborées à partir d'acide acétique et d'extraits de poudre de noix de coco, de solution de sels minéraux, d'extraits de cactacée, de liliacée, d'anacardiacée, et d'euphorbiacée.

2. Composition selon la revendication 1, caractérisée en ce qu'on utilise la chair blanche râpée de la noix de coco.

3. Composition selon la revendication 1 ou 2, caractérisée en ce qu'on utilise une opuntia, comme cactacée, en particulier de la raquette, et plus spécialement des fruits de la raquette verte.

4. Composition selon l'une des revendications 1 à 3, caractérisées en ce qu'on utilise, comme liliacée, de la salsepareille, en particulier de la racine de salsepareille.

5. Composition selon l'une des revendications 1 à 4, caractérisée en ce qu'on utilise, comme anacardiacée, du mombin, en particulier de l'écorce de mombin.

6. Composition selon l'une des revendications 1 à 5, caractérisée en ce qu'on utilise, comme euphorbiacée, des graines de ricin, en particulier de la poudre de graines de ricin grillées.

7. Composition selon l'une des revendications 1 à 6, caractérisée en ce qu'elle est élaborée à partir d'acide acétique, de noix de coco râpée, d'eau de mer, de fruits de raquette verte, de racine de salsepareille, d'écorce de mombin, de poudre de graines de ricin grillées.

8. Composition selon la revendication 7, caractérisée en ce qu'on utilise les différents ingrédients selon les proportions suivantes
- acide acétique: de 5 à 10 litres, notamment de 7 à 8 litres;
- poudre de noix de coco (de préférence, chair blanche râpée): équivalent d'environ 10 noix de coco ;
- solution de sels minéraux (notamment sodium, potassium, calcium, magnésium, phosphates, chlorures, sulfates, carbonates et bicarbonates), par exemple eau de mer : 3 à 5 litres, en particulier 4 litres ;
- raquette, notamment fruits de raquette verte râpés : de 10 à 20 fruits correspondant à 1 à 2 kg, notamment 15 fruits environ correspondant à environ 1,5 kg ;
- racine de salsepareille: 500 g à 1 kg, notamment 750 g environ ;
- écorce de mombin : de 500 g à 1,5 kg, en particulier environ 1 kg ;
- graines de ricin grillées et moulues : de 500g à 1,5 kg, notamment environ lkg.

9. Compositions possédant des propriétés antirétrovirales, caractérisées en ce qu'elles sont telles qu'obtenues en laissant macérer de la noix de coco en poudre avec de l'acide acétique, pendant environ 24 heures, en ajoutant au filtrat du mélange de macération les ingrédients selon l'une des revendications 1 à 8, avantageusement selon les proportions indiquées, en portant le mélange résultant à ébullition pendant au moins 1 heure, et en récupérant le filtrat.

10. Procédé d'obtention d'une composition selon l'une quelconque des revendications précédentes, caractérisé en ce qu'on fait macérer de la noix de coco en poudre avec de l'acide acétique, pendant environ 24 heures, on ajoute au filtrat du mélange de macération les ingrédients selon l'une des revendications 1 à 8, on porte le mélange à ébullition pendant au moins 1 heure et on récupère le filtrat.

11. Médicaments possédant notamment des propriétés antirétrovirales, caractérisés en ce qu'ils renferment une quantité efficace d'une composition selon l'une quelconque des revendications 1 à 9, en association avec un véhicule inerte.

## Patentansprüche

1. Stoffzusammensetzungen mit antiviralen und insbesondere antiretroviralen Eigenschaften, dadurch **gekennzeichnet,** daß sie zubereitet sind ausgehend von Essigsäure und Extrakten von gepulverter Kokosnuß, einer Lösung von anorganischen Salzen, und Extrakten von Cactacea, Liliacea, Anacardiacea und Euphorbiacea.

2. Stoffzusammensetzung nach Anspruch 1, dadurch **gekennzeichnet,** daß man das geraspelte weiße Fleisch der Kokosnuß verwendet.

3. Stoffzusammensetzung nach Anspruch 1 oder 2, dadurch **gekennzeichnet,** daß man als Cactacea eine Opuntia, besonders grünen Feigenkaktus und ganz besonders die Früchte des grünen Feigenkaktus verwendet.

4. Stoffzusammensetzung nach einem der Ansprüche 1 bis 3, dadurch **gekennzeichnet,** daß man als Liliacea die Salsaparille, besonders die Wurzel der Salsaparille verwendet.

5. Stoffzusammensetzung nach einem der Ansprüche 1 bis 4, dadurch **gekennzeichnet,** daß man als Anacardiacea Mombin (Spondias spec, Spondias purpurea, Spondias mombin), besonders die Rinde von Mombin verwendet.

6. Stoffzusammensetzung nach einem der Ansprüche 1 bis 5, dadurch **gekennzeichnet,** daß man als Euphorbiacea geröstete Rizinussamen, besonders Pulver von gerösteten Rizunussamen verwendet.

7. Stoffzusammensetzung nach einem der Ansprüche 1 bis 6, dadurch **gekennzeichnet,** daß sie zubereitet wird ausgehend von Essigsäure, geraspelter Kokosnuß, Meerwasser, Früchten von grünem Feigenkaktus, Wurzeln von Salsaparille, Rinde von Mombin und Pulver von gerösteten Rizinussamen.

8. Stoffzusammensetzung nach Anspruch 7, dadurch **gekennzeichnet**, daß man die verschiedenen Zutaten in den folgenden Anteilen verwendet :
- Essigsäure: 5 bis 10 Liter, besonders 7 bis 8 Liter;
- gepulverte Kokosnuß (vorzugsweise geraspeltes weißes Fleisch) : entsprechend etwa 10 Kokosnüssen;
- Lösung von anorganischen Salzen (Mineralsalzen) (besonders Phosphate, Chloride, Sulfate, Karbonate und Bikarbonate von Natrium, Kalium, Calcium, Magnesium), beispielsweise Meerwasser: 3 bis 5 Liter, besonders 4 Liter;
- grüner Feigenkaktus, besonders geraspelte Früchte von grünem Feigenkaktus ; 10 bis 20 Früchte entsprechend 1 bis 2 kg, besonders 15 Früchte entsprechend 1,5 kg;
- Salsaparillewurzel: 500 g bis 1 kg, besonders etwa 750 g;
- Mombinrinde: 500 g bis 1,5 kg, besonders etwa 1 kg;
- geröstete und gemahlene Rizinussamen: 500 g bis 1,5 kg, besonders etwa 1 kg.

9. Stoffzusammensetzungen mit antiretroviralen Eigenschaften, dadurch **gekennzeichnet,** daß sie solche sind, wie sie erhalten werden, indem man gepulverte Kokosnuß während etwa 24 Stunden mit Essigsäure mazeriert, dem Filtrat der Mazerationsmischung die Zutaten nach einem der Ansprüche 1 bis 8, vorzugsweise in den angegebenen Mengenanteilen zufügt, die erhaltene Mischung mindestens 1 Stunde zum Kochen bringt und das Filtrat gewinnt.

10. Verfahren zur Gewinnung einer Stoffzusammensetzung nach einem der vorangehenden Ansprüche, dadurch **gekennzeichnet,** daß man gepulverte Kokosnuß mit Essigsäure während etwa 24 Stunden mazeriert, dem Filtrat der Mazerationsmischung die Zutaten nach einem der Ansprüche 1 bis 8 zusetzt, das Gemisch während mindestens 1 Stunde zum Kochen bringt und das Filtrat gewinnt.

11. Arzneimittel, die insbesondere antiretrovirale Eigenschaften besitzen, dadurch **gekennzeichnet,** daß sie eine wirksame Menge einer Stoffzusammensetzung nach einem der Ansprüche 1 bis 9 zusammen mit einem inerten Trägermittel umfassen.

## Claims

1. Compositions having antiviral properties in particular and more specifically "antiretroviral" properties, characterized by the fact that they are elaborated from acetic acid and extracts of coconuts powder, mineral salts solution, extracts of cactacea, liliaceous, anacardiaceae and euphorbiaceae.

2. Composition according to claim 1, characterized by the fact that we use the grated white content of the coconut.

3. Composition according to claim 1 or 2, characterized by the fact that we use an opuntia, nopal in particular and more specifically the green nopal fruits as cactacea.

4. Composition according to one of the 1 to 3 claims, characterized by the fact that we use "salsepareille" and in particular root of "salsepareille" as liliaceous.

5. Composition according to one of the 1 to 4 claims, characterized by the fact that we use "mombin" and in particular bark of "mombin" as anacardiaceae.

6. Composition according to one of the 1 to 5 claims, characterized by the fact that we use castor oil plant seeds and in particular castor oil plant roasted and powdered seeds as euphorbiaceae.

7. Composition according to one of the 1 to 6 claims, characterized by the fact that it is elaborated from acetic acid, grated coconut, sea water, green nopal fruits, "salsepareille" root, "mombin" bark, castor oil plant powdered and roasted seeds.

8. Composition according to claim 8, characterized by the fact that we use the different ingredients according to the following proportions:
- acetic acid: from 5 to 10 liters and from 7 to 8 liters in particular;
- coconut powder (in particular the white grated content): equivalent to roughly 10 coconuts;
- mineral salts solution (sodium, potassium, calcium, magnesium, phosphates, chlorides, sulphates, carbonates and bicarbonates in particular), sea water, for example: from 3 to 5 liters and 4 liters in particular;
- nopal and grated fruits of green nopal in particular: from 10 to 20 fruits accounting for 1 to 2 kilos and around 15 fruits accounting for roughly 1.5 kg in particular;
- "salespareille" root: from 500 g to 1 kg and around 750 g in particular;
- bark of "mombin": from 500 g to 1.5 kg and around 1 kg in particular;
- ground and roasted seeds of castor oil plant: from 500 g to 1.5 kg and around 1 kg in particular.

9. Compositions having antiretroviral properties, characterized by the way we obtain them: we macerate coconuts in powder with acetic acid for roughly 24 hours and we add the ingredients to the filtrate according to one of the 1 to 8 claims and according to the indicated proportions. We then bring the mixture to the boil for at least 1 hour and we pick up the filtrate.

10. How to obtain one composition according to any previous claim, taking into account that we macerate coconuts in powder with acetic acid for roughly 24 hours and we add mixture of the macerated ingredients to the filtrate, according to claims 1 to 8 and we bring the mixture to the boil for at least 1 hour and we finally pick up the filtrate.

11. Drugs having "antiretroviral" properties, characterized by the fact that they possess a sufficient quantity of one composition according to any revendications from 1 to 9, associated to an inert vehicle.
